# EUROPÄISCHE PATENTSCHRIFT

(11) **EP 0 862 074 B1**
(45) Veröffentlichungstag und Bekanntmachung des Hinweises auf die Patenterteilung: **30.07.2003**
(21) Anmeldenummer: 97810104.6
(22) Anmeldetag: 26.02.1997
(51) Int. Cl.: G02C 9/00, G02C 7/16, A61F 9/02

(54) **Brille mit aufsetzbarem Schutzschild**
Spectacles with attachable protective shield
Lunettes avec écran de protection attachable

(43) Veröffentlichungstag der Anmeldung: 02.09.1998
(73) Patentinhaber: GRENDELMEIER, Alexander, CH-4663 Aarburg (CH)
(72) Erfinder: GRENDELMEIER, Alexander, CH-4663 Aarburg (CH)
(74) Vertreter: BOVARD AG - Patentanwälte

(56) Entgegenhaltungen:
- DE-U- 9 312 039
- FR-A- 2 224 121
- US-A- 5 428 407

## Beschreibung

Die vorliegende Erfindung bezieht sich auf eine Brille mit aufsetzbarem Schutzschild gemäss dem Oberbegriff des Patentanspruchs 1.

Brillen, bei denen zusätzliche Gläser beispielsweise als Sonnenschutz aufgesetzt werden können, sind bekannt. Diese Gläser weisen im wesentlichen die selbe Grösse auf, wie die Sehfehler korrigierenden Gläser der Brille und sind in einem Tragteil gehalten. An diesem Tragteil sind Bügel angebracht, so dass der Sonnenschutzteil auf die Brille aufgesteckt werden kann. Die Schutzgläser kommen dann auf die Sehfehler korrigierenden Gläser der Brille zu liegen, die Bügel stützen sich klemmend auf dem Hinterteil der Brillengläser bzw. des Gestells ab.

Die Halterung der vorgenannten Schutzgläser ist nicht optimal, insbesondere wenn die so ausgestattete Brille bei der Ausübung von Sportarten getragen werden soll. Zudem ist die Schutzfunktion dieser aufgesteckten Gläser nicht optimal, da sie üblicherweise lediglich die Grösse der Brillengläser aufweisen, wobei deren Grösse ja bekannterweise je nach Modetrend wechselt. Insbesondere bei kleinen Gläsern ist beispielsweise der Schutz vor Sonnenlicht ungenügend, die so ausgestatteten Brillen sind zudem auch als Windschutz für die Augen ungeeignet. Zusätzlich lässt bei derartigen Einrichtungen oftmals auch das ästhetische Erscheinungsbild einige Wünsche offen.

Aus der DE-U-93 12 039 ist eine Brille bekannt, auf welche ein Sonnenschutzglas aufgesetzt werden kann. Die Halterung besteht aus zwei Noppen, die in entsprechende Ausnehmungen eingeklipst werden können. Auch hierdurch ergibt sich keine optimale Verbindung.

Des weitern sind Sportbrillen bekannt, die bezüglich Sonnenschutz und Windschutz der Augen eine gute Wirkung haben. Diese Brillen weisen ein Schutzglas auf, das üblicherweise konvex gekrümmt ist, welches aber für Korrekturen von Sehfehlern nicht geeignet ist. Hierzu können in diese Brillen Einsätze eingesetzt werden, die die entsprechenden Korrekturgläser aufweisen. Diese Brillen sind für die Ausübung von Sportarten gut geeignet, der Sonnenschutz und der Windschutz sind optimal. Wenn beispielsweise aber eine Person, die eine derartige Sonnenschutzbrille trägt, sich vom Sonnenlicht in einen geschlossenen Raum begibt, kann mit dieser Sonnenbrille nichts gesehen werden, weil es im Raum zu dunkel ist. Somit muss diese Sonnenbrille mit dem korrigierenden Einsatz abgenommen werden und durch eine normale Sehbrille ersetzt werden. Man ist somit gezwungen, zusätzlich zur Sonnenbrille mit dem Korrektureinsatz eine zweite normale Brille mitzutragen. Da eine Schutzbrille mit Sehfehler korrigierendem Einsatz relativ teuer ist, ist das Anschaffen einer derartigen zusätzlichen Sportbrille eine Kostenfrage.

Die Aufgabe der vorliegenden Erfindung besteht nun darin, eine Brille mit aufsetzbarem Schutzschild zu schaffen, die eine optimale Schutzwirkung beispielsweise gegen Sonnenlicht oder Wind bietet, wobei ein einfaches Aufsetzen des Schutzschildes auf die Brille möglich sein soll und die Verbindung zwischen Brille und Schutzschild optimal ist.

Erfindungsgemäss erfolgt die Lösung dieser Aufgabe durch die im Anspruch 1 aufgeführten Merkmale.

Des weitern soll diese Brille, die für sich allein oder mit aufgesetztem Schutzschild getragen werden kann, die von einem Träger gestellten Anforderungen an ein ästhetisches Erscheinungsbild erfüllen.

Zum Erreichen einer optimalen Fixierung des Schutzschildes auf der Brille bestehen die Haltemittel aus zwei seitlich angebrachten Stiften und einem zentralen Stift, die in den entsprechenden schlitzförmigen Ausnehmungen, die am Brillengestell angebracht sind, gehalten sind.

Zum Aufsetzen des Schutzschildes auf die Brille bzw. zum Einsetzen der Stifte in die vorgesehenen Ausnehmungen am Brillengestell wird der Schutzschild, nachdem die beiden seitlichen Stifte in den entsprechenden Ausnehmungen eingeführt worden sind, im mittleren Bereich gegen das Brillengestell gedrückt, wobei eine elastische Verformung statt findet, und der zentrale Stift wird in die zentrale Ausnehmung eingeführt. Beim Zurückfedem verhängt sich die Verdickung des zentralen Stiftes in einer entsprechenden Erweiterung der zentralen schlitzförmigen Ausnehmung, der Sitz des Schutzschildes auf der Brille ist somit praktisch spielfrei. Beim Tragen der Brille durch eine Person wird die auf den zentralen Stift wirkende Haltekraft noch verstärkt.

In vorteilhafter Weise sind die beiden seitlichen Stifte und der zentrale Stift jeweils in mit Gewinde versehene Bohrungen, die am Schutzschild angebracht sind, eingeschraubt. Hierzu weisen die Stifte neben einem Einschraubteil einen flanschförmigen Abstützteil auf, an welchem jeweils an der dem Schutzschild abgewandten Seite zwei Bohrungen angebracht sind. Diese Bohrungen dienen als Angriffspunkte für ein entsprechend gestaltetes Werkzeug, wodurch die Stifte problemlos in den Schutzschild eingeschraubt werden können.

Wenn der Schutzschild als Schutz gegen die Sonne verwendet werden soll, wird er entsprechend farblich getönt. Es ist ohne weiteres auch möglich, den Schutzschild aus klarsichtigem Material herzustellen und auf die Brille aufzusetzen, wodurch man eine Schutzbrille erhält, die als mechanischer Schutz dient und beispielsweise bei spanender Bearbeitung von Werkstücken die Augen vor herumfliegenden Spänen schützt.

Weitere vorteilhafte Ausgestaltungen der Erfindung ergeben sich aus den weiteren abhängigen Ansprüchen.

Eine Ausführungsform einer erfindungsgemässen Brille mit aufsetzbarem Schutzschild wird nachfolgend anhand der beiliegenden Zeichnung beispielhaft näher erläutert.

Es zeigt:
Fig. 1 eine Ansicht von vorn auf eine erfindungsgemässe Brille mit aufgesetztem Schutzschild;
Fig. 2 eine Darstellung der zentralen schlitzförmigen Ausnehmung im Brillengestell;
Fig. 3 eine Ansicht des zentralen Stiftes;
Fig. 4 eine Draufsicht auf den zentralen Stift gemäss Fig. 3;
Fig. 5 eine Darstellung einer seitlichen schlitzförmigen Ausnehmung im Brillengestell;
Fig. 6 eine Schnittdarstellung durch das Brillengestell entlang Linie VI-VI gemäss Fig. 5 mit aufgesetztem Schutzschild;
Fig. 7 eine Draufsicht auf die erfindungsgemässe Brille mit aufgesetztem Schutzschild; und
Fig. 8 eine Draufsicht auf die Brille gemäss Fig. 7 mit abgenommenem Schutzschild.

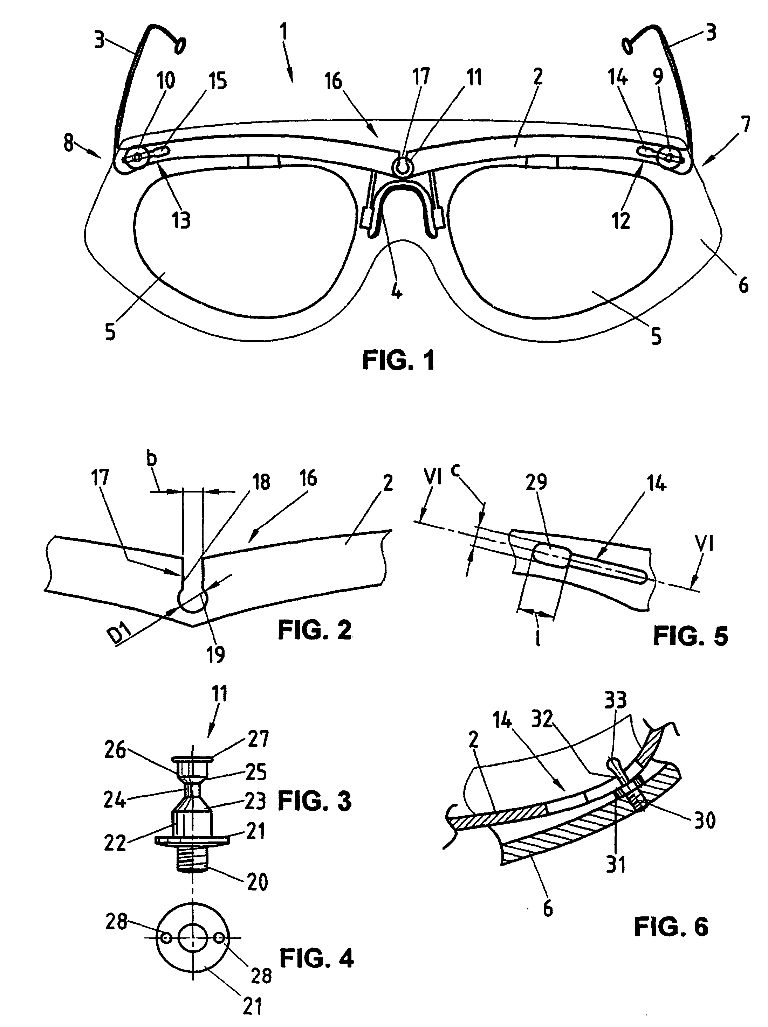

In Fig. 1 ist eine Brille 1 dargestellt, die ein Brillengestell 2 mit angelenkten Bügeln, einem Nasenbügel 4 und Sehfehler korrigierenden Brillengläser 5 umfasst. Die Brillengläser 5 können hierbei mit dem Brillengestell 2 lösbar und austauschbar verbunden sein, wie dies beispielsweise in der EP-A-0 514 514 beschrieben ist.

Auf die Brille 1 aufgesetzt ist ein Schutzschild 6. Dieses Schutzschild 6 besteht beispielsweise aus einem Acrylglas, das klarsichtig oder getönt sein kann, je nach dem ob dieser Schutzschild 6 als Schutz der Augen gegen herumfliegende Gegenstände, beispielsweise Späne bei spanender Bearbeitung von Werkstücken, als Windschutz bei trübem Wetter oder als zusätzlicher Sonnenschutz verwendet wird. In diesen Schutzschild 6 sind jeweils an einem seitlichen Randbereich 7, 8 je ein Stift 9 bzw. 10 eingesetzt. Etwa in der Mitte zwischen diesen beiden Stiften 9 und 10 ist ein zentraler Stift 11 in den Schutzschild 6 eingesetzt.

Im Brillengestell 2 ist an den seitlichen Randbereichen 12 und 13 je eine seitliche schlitzförmige Ausnehmung 14 bzw. 15 angebracht, während im mittleren Bereich 16 des Brillengestells 2 eine zentrale schlitzförmige Ausnehmung 17 vorgesehen ist. Wenn der Schutzschild 6 auf die Brille 1 aufgesetzt ist, sind die Stifte 9 bzw. 10 in den entsprechenden seitlichen schlitzförmigen Ausnehmungen 14 bzw. 15 gehalten, während der zentrale Stift 11 in der zentralen schlitzförmigen Ausnehmung 17 des Brillengestells 2 gehalten ist, wie nachfolgend noch genauer beschrieben wird.

In Fig. 2 ist der mittlere Bereich 16 des Brillengestells 2 dargestellt. Hierbei ist die zentrale schlitzförmige Ausnehmung 17 ersichtlich. Diese zentrale schlitzförmige Ausnehmung 17 verläuft im wesentlichen quer zum Brillengestell 2. Die zentrale schlitzförmige Ausnehmung 17, die gegen den oberen Rand des Brillengestells 2 hin offen ist, weist eine Verengung 18 und eine an die Verengung anschliessende Bohrung 19 auf. Die Verengung 18 hat eine Breite b, während der Durchmesser der Bohrung 19 D1 ist.

In die zentrale schlitzförmige Ausnehmung 17 des Brillengestells 2 kommt im aufgesetzten Zustand des Schutzschildes 6 der zentrale Stift 11 zu liegen, der in Fig. 3 und 4 dargestellt ist. Dieser zentrale Stift 11 weist einen Einschraubteil 20, der in den Schutzschild 6 einschraubbar ist, einen flanschförmigen Abstützteil 21, einen ersten zylindrischen Teil 22, einen sich verjüngenden ersten Kegelstumpf 23, einen zweiten zylindrischen Teil 24, einen sich erweiternden zweiten Kegelstumpf 25 und einen daran anschliessenden dritten zylindrischen Teil 26 auf, der mit einem Kragen 27 abgeschlossen ist. Wie aus Fig. 4 ersichtlich ist, sind am flanschförmigen Abstützteil 21 zwei Bohrungen 28 angebracht, in welche ein entsprechend gestaltetes Werkzeug einsetzbar ist, wodurch der zentrale Stift 11 in den Schutzschild 6 eingeschraubt werden kann.

Beim Aufsetzen des Schutzschildes 6 auf die Brille 1 wird der zentrale Stift 11 mit seinem zweiten zylindrischen Teil 24, dessen Durchmesser kleiner ist als die Breite b der Verengung 18 bis in die Bohrung 19 der zentralen schlitzförmigen Ausnehmung 17 eingesetzt. Der erste Kegelstumpf 23 und der zweite Kegelstumpf 25 dienen hierbei als Einführhilfen. Wie später noch gesehen wird, federt der Schutzschild 6 im auf die Brille 1 aufgesetzten Zustand vom Brillengestell 2 weg, so dass der dritte zylindrische Teil 26 des zentralen Stiftes 11 in die Bohrung 19 der zentralen schlitzförmigen Ausnehmung 17 gezogen wird, bis sich der Kragen 27 auf der Oberfläche des Brillengestells 2 abstützt. Der zentrale Stift 11 ist somit sicher in der zentralen schlitzförmigen Ausnehmung 17 gehalten.

Wie aus Fig. 5 ersichtlich ist, weisen die seitlichen schlitzförmigen Ausnehmungen 14 bzw. 15, die in den seitlichen Randbereichen 12 und 13 des Brillengestells 2 eingelassen sind, Erweiterungen 29 auf, wobei nur die seitliche schlitzförmige Ausnehmung 14 gezeigt ist, während die seitliche schlitzförmige Ausnehmung 15 spiegelbildlich angeordnet ist. Diese Erweiterung 29 hat jeweils eine Breite c und eine Länge l.

In diese seitliche schlitzförmige Ausnehmung 14 kommt der am Schutzschild 6 angebrachte Stift 9 zu liegen, wenn der Schutzschild 6 auf die Brille 1 aufgesetzt ist, wie in Fig. 6 dargestellt ist. Der Stift 9 setzt sich aus einem Einschraubteil 30, einem flanschförmigen Abstützteil 31 und einem zylindrischen Teil 32 zusammen, der mit einer kugelförmigen Verdickung 33 abgeschlossen ist. Auch hier weist der flanschförmige Abstützteil 31 Bohrungen auf, die den Bohrungen 28 des zentralen Stiftes 11 entsprechen, dargestellt in Fig. 4. Damit lässt sich ebenfalls der Stift 9 in den Schutzschild 6 einschrauben. Entsprechend ist der Stift 10 ausgebildet, der in die schlitzförmige Ausnehmung 15 zu liegen kommt.

Beim Aufsetzen des Schutzschildes 6 auf die Brille 1 wird die Verdickung 33 durch die Erweiterung 29 der seitlichen schlitzförmigen Ausnehmung 14 geführt, wobei gemäss Fig. 5 die Breite c geringfügig und die Länge I grösser ist als die Verdickung 33 des Stiftes 9. Im aufgesetzten Zustand des Schutzschildes 6 auf die Brille 1 befindet sich der Stift 9 mit seinem zylindrischen Teil 32 im an die Erweiterung 29 anschliessenden Bereich der seitlichen schlitzförmigen Ausnehmung 14, welche eine Breite aufweist, die kleiner ist als der Durchmesser der Verdickung 33. Somit wird auch der seitliche Bereich des Schutzschildes 6 optimal an der Brille 1 gehalten. Es versteht sich von selbst, dass der zweite Stift 10 auf der anderen Seite des Schutzschildes 6 entsprechend in die seitliche schlitzförmige Ausnehmung 15 eingesetzt wird und darin gehalten ist.

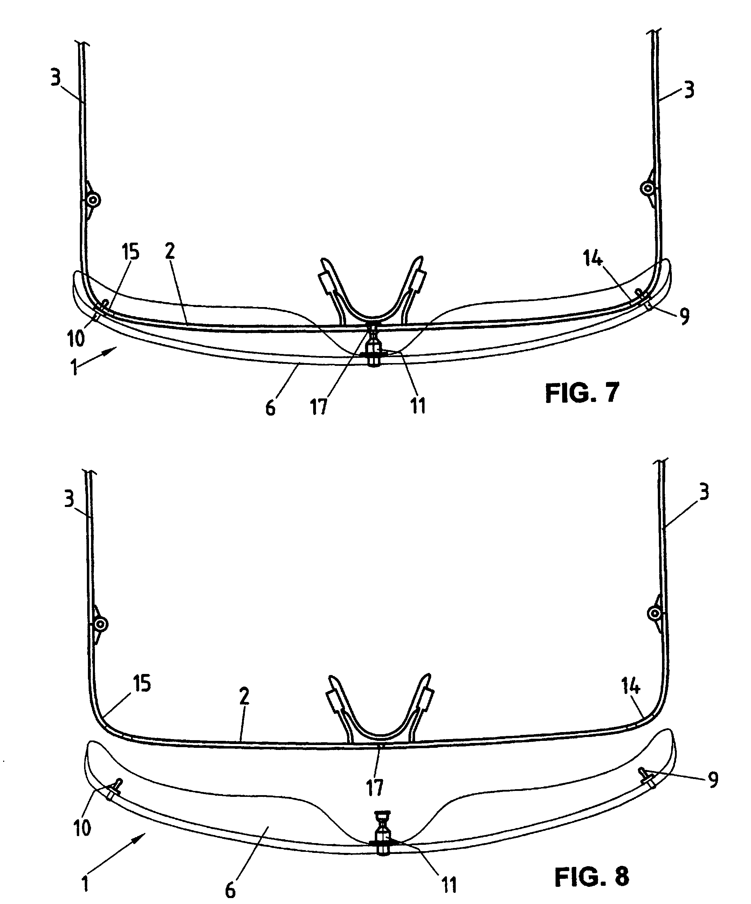

Zum Aufsetzen des Schutzschildes 6 auf die Brille 1 wird, wie aus Fig. 8 ersichtlich ist, der Schutzschild 6 gegen das Brillengestell 2 geführt, derart dass die Stifte 9 und 10 in die seitlichen schlitzförmigen Ausnehmungen 14 und 15 eindringen können, wie zu Fig. 5 und 6 beschrieben worden ist. Danach wird der Schutzschild 6, dessen Krümmung etwas grösser ist als diejenige des Brillengestells 2 im mittleren Bereich gegen das Brillengestell 2 gedrückt, bis der zweite zylindrische Teil 22 des zentralen Stiftes 11 oberhalb der zentralen schlitzförmigen Ausnehmung 17 zu stehen kommt. Danach wird das Brillengestell 2 und der Schutzschild 6 zueinander verschoben, so dass der zentrale Stift 11 quer zur Längsachse in die Bohrung 19 der zentralen schlitzförmigen Ausnehmung 17 eingeführt wird. Danach wird beim Loslassen der Schutzschild 6 zurückfedern, so dass der dritte zylindrische Teil 26 des zentralen Stiftes 11 in die Bohrung 19 gezogen wird, bis der Kragen 27 am Brillengestell 2 anliegt, wie dies zu den Fig. 2 bis 4 bereits beschrieben worden ist. Die Brille 1 mit aufgesetztem Schutzschild 6 ist in Fig. 7 dargestellt. Durch das gegeneinander Drücken des Schutzschildes 6 und des Brillengestells 2 verschieben sich die Stifte 9 und 10 in den seitlichen schlitzförmigen Ausnehmungen 14 und 15 gegen aussen, sie gelangen somit in den Bereich, dessen Breite kleiner ist als der Durchmesser der Verdickung 33 der Stifte 9 und 10, wie dies in den Fig. 5 und 6 dargestellt ist. Somit sind auch die Stifte 9 und 10 optimal am Brillengestell 2 gehalten. Durch das elastische Verspannen des Schutzschildes 6 gegen das Brillengestell 2 hin stützt sich der Schutzschild 6 mit den flanschförmigen Abstützteilen 31 der Stifte 9 und 10 auf dem Brillengestell 2 ab, wodurch eine spielfreie Halterung des Schutzschildes 6 auf der Brille 1 erreicht wird.

Zum Abnehmen des Schutzschildes 6 von der Brille 1 wird der Vorgang, wie er vorhin beschrieben worden ist, in umgekehrter Reihenfolge durchgeführt.

Mit dieser erfindungsgemässen Brille mit aufsetzbarem Schutzschild wird ein optimaler Schutz der Augen ermöglicht, wobei die Sehfehler korrigierenden Brillengläser der Brille mitverwendet werden. Eine derartige Brille bietet Schutz gegen Sonnenlicht, aber auch in optimaler Weise gegen herumfliegende Spritzer oder Gegenstände, beispielsweise von Spänen, aber auch gegen Fahrtwind, wobei wegen der Brillengläser eine optimale Sicht gewährleistet ist. Der Schutzschild kann auch mit Ausnehmungen versehen sein, so dass er über Fernrohrlupen, die beispielsweise auf die Brillengläser aufgeklebt werden können, geführt werden kann. Dies bedeutet, dass eine derartige Brille mit aufsetzbarem Schutzschild insbesondere auch für Medizinalberufe wie Ärzte, Zahnärzte und ähnliche geeignet ist. Dabei kann der Schutzschild so gestaltet sein, dass er optimal zu einem Mund- und Nasenschutz passt.

Der Schutzschild kann problemlos auf die entsprechende Brille aufgesetzt oder von dieser wieder abgenommen werden, wobei die Brille in optimaler Weise die Funktion einer reinen Sehbrille erfüllt. Sowohl bei aufgesetztem Schutzschild wie auch bei abgenommenem Schutzschild wirkt die Brille in ästhetischer Hinsicht optimal.

## Patentansprüche

1. Brille mit aufsetzbarem Schutzschild (6), bestehend aus einem Brillengestell (2), mit im Brillengestell (2) gehaltenen, Sehfehler korrigierenden Brillengläsern (5), wobei am aufsetzbaren Schutzschild (6) und/oder am Brillengestell (2) Haltemittel angeordnet sind, die zwei Stifte (9; 11) umfassen, die in den Schutzschild (6) eingesetzt und vorstehend sind und am dem Schutzschild (6) abgewandten Bereich mit einer Verdickung (33; 26, 27) ausgestattet sind, und am Brillengestell (2) entsprechend den am Schutzschild (6) eingesetzten Stiften (9; 11) zwei schlitzförmige Ausnehmungen (14; 17) angebracht sind, mit welchen der Schutzschild (6) am Brillengestell (2) vor den Brillengläsern (5) und im wesentlichen parallel dazu gehalten ist, **dadurch gekennzeichnet, dass** je einer der beiden Stifte (9, 10) an jeweils einem seitlichen Randbereich (7 bzw. 8) in den Schutzschild (6) eingesetzt ist und im wesentlichen zwischen diesen beiden Stiften (9, 10) ein zentraler Stift (11) in den Schutzschild (6) eingesetzt ist, dass entsprechend an den beiden seitlichen Randbereichen (12, 13) des Brillengestells (2) je eine seitliche schlitzförmige Ausnehmung (14, 15) und im mittleren Bereich (16) eine zentrale schlitzförmige Ausnehmung (17), die jeweils eine Erweiterung aufweisen, in das Brillengestell eingelassen sind, und die Längsachsen der beiden seitlichen schlitzförmigen Ausnehmungen (14, 15) und deren zugehörige Erweiterungen (29)im wesentlichen gegeneinander gerichtet sind, dass die zentrale schlitzförmige (17) Ausnehmung im wesentlichen quer zu den genannten beiden schlitzförmigen Ausnehmungen (14, 15) angebracht ist, so dass das Brillengestell (2) und der aufgesetzte Schutzschild (6) gegeneinander elastisch verspannt werden.

2. Brille mit aufsetzbarem Schutzschild nach Anspruch 1, **dadurch gekennzeichnet, dass** der Schutzschild (6) eine konvex gewölbte Oberfläche aufweist, die im auf die Brille (1) aufgesetzten Zustand gegen aussen gerichtet ist, und dass die Stifte (9,10,11) jeweils senkrecht zu dieser Oberfläche stehen und gegen innen gerichtet sind.

3. Brille mit aufsetzbarem Schutzschild nach Anspruch 1 oder 2, **dadurch gekennzeichnet, dass** die beiden an den seitlichen Randbereichen (7, 8) befestigten Stifte (9, 10) je aus einem Einschraubteil (30), einem flanschförmigen Abstützteil (31) und einem zylindrischen Teil (32) bestehen und die an den zylindrischen Teil (32) anschliessende Verdickung (33) kugelförmig ist, und dass die entsprechende schlitzförmige Ausnehmung (14, 15) eine Breite aufweist, die kleiner ist als die kugelförmige Verdickung, während die Breite (c) der Erweiterung (29) geringfügig grösser und die Länge (I) der Erweiterung (29) grösser ist als der Durchmesser der kugelförmigen Verdickung (33).

4. Brille mit aufsetzbarem Schutzschild nach Anspruch 3, **dadurch gekennzeichnet, dass** im flanschförmigen Abstützteil (31) der beiden an den seitlichen Randbereichen (7, 8) des Schutzschildes (6) befestigten Stiften (9, 10) an der dem Schutzschild (6) abgewandten Seite jeweils zwei Bohrungen angebracht sind.

5. Brille mit aufsetzbarem Schutzschild nach einem der Ansprüche 1 bis 4, **dadurch gekennzeichnet, dass** der zentrale Stift (11) aus einem Einschraubteil (20), einem flanschförmigen Abstützteil (21), einem ersten zylindrischen Teil (22), einem sich verjüngendem ersten Kegelstumpf (23), einem zweiten zylindrischen Teil (24), einem sich erweiternden zweiten Kegelstumpf (25) und einem daran anschliessenden dritten zylindrischen Teil (26) besteht, der mit einem Kragen (27) abgeschlossen ist, und dass die entsprechende zentrale schlitzförmige Ausnehmung (17) gegen den oberen Rand des Brillengestells (2) hin trichterförmig geöffnet ist, eine Verengung (18) und eine an die Verengung (18) anschliessende Bohrung (19) aufweist, wobei die Breite (b) der Verengung (18) geringfügig grösser ist, als der Durchmesser des zweiten zylindrischen Teils (24), und der Durchmesser (D1) der Bohrung etwa dem Durchmesser des dritten zylindrischen Teils (26) entspricht.

6. Brille mit aufsetzbarem Schutzschild nach Anspruch 5, **dadurch gekennzeichnet, dass** im flanschförmigen Abstützteil (21) des zentralen, am Schutzschild (6) befestigten Stiftes (11) an der dem Schutzschild (6) abgewandten Seite zwei Bohrungen (28) angebracht sind.

7. Brille mit aufsetzbarem Schutzschild nach einem der Ansprüche 1 bis 6, **dadurch gekennzeichnet, dass** der Schutzschild (6) aus Acrylglas besteht, das kiarsichtig oder farblich getont ist.

## Claims

1. Eyeglasses with attachable protective shield consisting of an eyeglass frame (2) with prescription lenses (5) held in the frame (2), holding means being disposed on the attachable protective shield (6) and/or on the eyeglass frame (2), which holding means comprise two pins (9: 11), which are inserted into the protective shield (6) and are protruding and which are provided with a swelling (33; 26, 27) on the area turned away from the protective shield (6), and two slot-shaped recesses (14; 17) are provided in the frame (2) corresponding to the pins (9; 11) inserted on the protective shield (6), with which recesses the protective shield (6) is held on the eyeglass frame (2) in front of the lenses (5) and essentially parallel thereto, **characterised in that** one of the two pins (9, 10) in each case is inserted in the protective shield (6) in a lateral edge area (7 or respectively 8) in each case, and a central pin (11) is inserted in the protective shield (6) substantially between these two pins (9, 10), **in that** provided correspondingly in the eyeglass frame on the two lateral edge areas (12, 13) of the eyeglass frame (2) are one lateral slot-shaped recess (14, 15) each and in the middle area (16) a central slot-shaped recess (17) which each have an extension, and the longitudinal axes of the two lateral, slot-shaped recesses (14, 15) and their respective extensions (29) are directed substantially toward one another, and **in that** the central, slot-shaped recess (17) is disposed substantially transversely to the said two lateral slot-shaped recesses (14, 15), so that the eyeglass frame (2) and the attached protective shield (6) become elastically tensioned with respect to each other.

2. Eyeglasses with attachable protective shield according to claim 1, **characterised in that** the protective shield (6) has a curved, convex surface, which, when in the state attached to the eyeglasses (1), is directed outward, and **in that** the pins (9, 10, 11) each stand perpendicular to this surface and are directed inward.

3. Eyeglasses with attachable protective shield according to claim 1 or 2, **characterised in that** the two pins (9, 10) mounted on the lateral edge areas (7, 8) each consist of a screw portion (30), a flange-shaped supporting portion (31) and a cylindrical portion (32), and the swelling (33) adjoining the cylindrical portion (32) is spherical, and **in that** the corresponding slot-shaped recess (14, 15) has a width which is smaller than the spherical thickness, whereas the width (c) of the extension (29) is slightly larger and the length (I) of the extension (29) is greater than the diameter of the spherical swelling (33).

4. Eyeglasses with attachable protective shield according to claim 3, **characterised in that** two bores each are made in the flange-shaped supporting part (31) of the two pins (9, 10), mounted on the lateral edge areas (7, 8) of the protective shield (6), on the side turned away from the protective shield (6).

5. Eyeglasses with attachable protective shield according to one of the claims 1 to 4, **characterised in that** the central pin (11) consists of a screw part (20), a flange-shaped supporting part (21), a first cylindrical part (22), a tapering first frustum (23), a second cylindrical part (24), a widening second frustum (25) and an adjoining third cylindrical part (26), which is terminated with a collar (27), and **in that** the corresponding central slot-shaped recess (17) is opened in a funnel-shape toward the upper edge of the eyeglass frame (2), has a narrowing (18) and a bore (19) adjoining the narrowing (18), the width (b) of the narrowing (18) being slightly larger than the diameter of the second cylindrical part (24), and the diameter (D1) of the bore corresponding approximately to the diameter of the third cylindrical part (26).

6. Eyeglasses with attachable protective shield according to claim 5, **characterised in that** two bores (28) are made in the flange-shaped supporting part (21) of the central pin (11), fixed to the protective shield (6), on the side turned away from the protective shield (6).

7. Eyeglasses with attachable protective shield according to one of the claims 1 to 6, **characterised in that** the protective shield (6) is made of acrylic glass which is clear or tinted.

## Revendications

1. Lunette avec visière de protection amovible (6), se composant d'une monture (2), de verres de lunette (5) correcteurs et maintenus dans la monture (2), des moyens de retenue étant disposés sur la visière de protection amovible (6) et/ou sur la monture de lunette (2), lesquels comprennent deux tiges (9, 11) qui sont insérées dans la visière de protection (6), qui en font saillie et qui sont munies sur la zone détournée de la visière de protection (6) d'un renflement (33, 26, 27), et deux évidements en forme de fente (14, 17) étant ménagés sur la monture (2) en fonction des tiges (9, 11) insérées sur la visière de protection (6), évidements avec lesquels la visière de protection (6) est maintenue sur la monture (2) sensiblement parallèlement devant les verres de lunette (5), **caractérisée en ce que** chacune des deux tiges (9, 10) est insérée dans la visière de protection (6) sur une zone de bordure latérale respective (7 respectivement 8) et **en ce qu'**une tige centrale (11) est introduite sensiblement entre les deux tiges (9, 10) dans la visière de protection (6), **en ce qu'**il est réalisé dans la monture en conséquence sur les deux zones de bordure latérale (12, 13) de la monture (2) respectivement un évidement latéral (14, 15) en forme de fente et dans la zone médiane (16) un évidement central (17) en forme de fente, et **en ce que** les axes longitudinaux des deux évidements latéraux en forme de fente (14, 15) et leurs élargissements respectifs (29) sont dirigés sensiblement les uns vers les autres, **en ce que** l'évidement central en forme de fente (17) est réalisé sensiblement transversalement auxdits deux évidements latéraux en forme de fente (14, 15), de sorte que la monture (2) et la visière de protection (6) posée sont serrées de manière élastique l'une contre l'autre.

2. Lunette avec visière amovible de protection, selon la revendication 1, **caractérisée en ce que** la visière de protection (6) présente une surface bombée convexe qui est dirigée vers l'extérieur à l'état monté sur la lunette (1) et **en ce que** les tiges (9, 10, 11) sont placées respectivement perpendiculairement à cette surface et dirigées vers l'intérieur.

3. Lunette avec visière amovible de protection, selon la revendication 1, **caractérisée en ce que** les deux broches (9, 10) fixées sur les zones latérales de bordure (7, 8) se composent chacune d'une partie filetée (30), d'une partie d'appui en forme de bride (31) et d'une partie cylindrique (32) et le renflement (33) situé dans le prolongement de la partie cylindrique (32) présente une forme sphérique et **en ce que** l'évidement correspondant en forme de fente (14, 15) présente une largeur qui est inférieure au renflement sphérique tandis que la largeur (c) de l'élargissement (29) est légèrement supérieure et la longueur (I) de l'élargissement (29) est légèrement supérieure au diamètre du renflement sphérique (33).

4. Lunette avec visière amovible de protection, selon la revendication 3, **caractérisée en ce que** deux perçages sont ménagés respectivement sur la face détournée de la visière de protection (6) dans la partie d'appui en forme de bride (31) des deux tiges (9, 10) fixées sur les zones de bordure latérales (7, 8) de la visière de protection (6).

5. Lunette avec visière amovible de protection, selon l'une des revendications 1 à 4, **caractérisée en ce que** la tige centrale (11) se compose d'une partie filetée (20), d'une partie d'appui en forme de bride (21), d'une première partie cylindrique (22), d'un premier tronc conique effilé (23), d'une seconde partie cylindrique (24) et d'un second tronconique en entonnoir (25) et, dans le prolongement, d'une troisième partie cylindrique (26) qui est terminée par une collerette (27) et **en ce que** l'évidement central correspondant en forme de fente (17) est ouvert en forme d'entonnoir dirigé vers le bord supérieur de la monture (2), présente un étranglement (18) et un perçage (19) se trouvant dans la continuité de l'étranglement (18), la largueur (b) de l'étranglement (18) étant légèrement supérieure au diamètre de la seconde partie cylindrique (24) et **en ce que** le diamètre (D1) du perçage correspond approximativement au diamètre de la troisième partie cylindrique (26).

6. Lunette avec visière amovible de protection, selon la revendication 5, **caractérisée en ce que** deux perçages (28) sont ménagés sur la face détournée de la visière de protection (6) dans la partie d'appui en forme de bride (21) de la tige centrale (11) fixée sur la visière de protection (6).

7. Lunette avec visière amovible de protection, selon l'une des revendications 7 à 6, **caractérisée en ce que** la visière de protection (6) se compose de verre acrylique qui est transparent ou teinté.
